Europäisches Patentamt

European Patent Office

Office européen des brevets

(11) Publication number: **0 085 362
B1**

# EUROPEAN PATENT SPECIFICATION

(45) Date of publication of patent specification: **16.09.87**

(51) Int. Cl.⁴: **G 01 N 33/86**

(21) Application number: **83100483.3**

(22) Date of filing: **20.01.83**

(54) Method for determining allergic sensitivity.

(30) Priority: **01.02.82 US 344478**

(43) Date of publication of application:
**10.08.83 Bulletin 83/32**

(45) Publication of the grant of the patent:
**16.09.87 Bulletin 87/38**

(84) Designated Contracting States:
**AT BE CH DE FR GB IT LI LU NL SE**

(56) References cited:
**FR-A-2 327 548
US-A-3 486 981
CHEMICAL ABSTRACTS, vol. 68, no. 7, 12th
February 1968, Columbus, Ohio, USA; W.O.
CRUZ et al. "Antihemostatic effect of heparin
counteracted by adenosine triphosphate",
page 2685, column 2, abstract no. 27870z**
**THE JOURNAL OF IMMUNOLOGY, vol. 123, no.
6, December 1979, Baltimore, USA; L.M.
McMANUS et al. "Platelet activating factor
(PAF) induced release of platelet factor 4 (PF4)
in vitro and during IgE anaphylaxis in the
rabbit", pages 2835-2841**

(73) Proprietor: **MILES LABORATORIES, INC.
1127 Myrtle Street
Elkhart Indiana 46514 (US)**

(72) Inventor: **Hammond, Michael Douglas
3 London Road
Postcombe Oxon (GB)**
Inventor: **Taylor, William James Arthur
'Wyndin' The Willows Maidenhead Road
Windsor, Berks (GB)**

(74) Representative: **Jesse, Ralf-Rüdiger, Dr. et al
Bayer AG Konzernverwaltung RP
Patentabteilung
D-5090 Leverkusen 1 Bayerwerk (DE)**

(56) References cited:
**CLINICAL RESEARCH, vol. 25, 1977; A.P.
KAPLAN et al. "Human Anaphylaxis: A study of
mediator systems", page 361A, column 2,
paragraph 2**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

## Description

Background of the invention

1. Field of the invention

The basic function of the organs, cells and molecules that comprise the immune system is to recognize and to eliminate from the body foreign substances. These foreign substances are eliminated by reaction between the foreign substance and antibodies which are formed in response to the substance. In general, this function is performed efficiently and without detriment to the host. However, in hypersensitive individuals, disturbances can occur which can lead to pathogenic disorders such as, an uncontrolled response, for example, (allergic disorders).

Hypersensitive individuals undergo an "altered state" as a result of contact with the antigens from an allergen, leading to the formation of antibodies thereto. Subsequent contact with one of those antigens or a structurally similar substance can evoke in a hypersensitive individual a pathological reaction, due to the presence of such antibodies. When these individuals inhale or ingest the offending antigen, a prominent and common manifestation includes hay fever, asthma, urticaria ("hives") or dermatitis. The tendency to develop this form of allergy is hereditable.

Allergic responses are involved with the production within an individual of tissue-sensitizing IgE antibodies. These IgE antibodies have a high affinity for receptors on cells present in various body tissues. The receptors are on mast cells which are found in close association with capillaries in connective tissues throughout the body and on basophilic leukocytes (blood cells). Mast cells and basophils contain pharmacologically-active mediators or spasmogens, such as histamine, serotonin (5)hydroxytryptamine) and kinins (basic peptides), concentrated in cytoplasmic granules. Contact of the IgE antibodies, which are fixed to mast cells and basophils, with antigens can trigger cross-linking of the IgE antibodies. In turn, this cross-linking causes degranulation of mast cells and basophils, which releases the chemical mediators and produces manifestations of the allergic response referred to earlier.

Allergy diagnosis can be accomplished in numerous ways. A frequently used method invokes studying the patient's history, i.e., recent exposure to various allergens, and based on a decision regarding the identity of the suspected allergen, injecting small amounts of the suspected allergen into the skin, and examining the injected site for a reaction which is characterized by erythema and wheal formation and associated pruritus. This method is quick and easy for the clinician and considered to be a reliable indication of allergen sensitivity because it is conducted *in vivo*. However, this method can involve considerable patient discomfort, is difficult to conduct with children, can expose an extremely hypersensitive patient to danger, and may be difficult to interpret in patients with severe dermatitis.

When allergy to foods is suspected, dietary exclusion tests can be utilized. These tests are not convenient for the patient and can involve a lengthy procedure.

*In vitro* tests which involve specific binding assay methods are also used. A typical specific binding assay procedure is the determination of IgE antibodies in the blood of a hypersensitive individual. U.S. Patent No. 3,720,760 is directed to a technique which measures allergen-specific IgE levels in serum by the radioallergosorbent test (RAST). This type of *in vitro* test is expensive and requires a sophisticated laboratory in addition to requiring about 36 hours to complete.

Description of the prior art

*J. Immunol.*, *115*: 525—532 (1975) describes a study involving rabbits, of IgE-induced blood coagulation alterations after antigen challenge, and its initiation of blood coagulation. The research involved demonstrated a decrease in the functional levels of blood clotting factors XII, XI, and IX and in the clottable fibrinogen concentration (Factor I) and a prolongation of the partial thromboplastin time, i.e., an increase in the time required for blood coagulation. The authors state that the study is the first reported documentation of the involvement of specific IgE antibody in the initiation of blood coagulation.

*Clin. Res.*, *25*: 361A (1977) describes a study of immunotherapy for insect hypersensitivity in man. The study involved 20 patients, sensitive to insect venom. The patients were challenged with increasing doses of venom. There were 3 cases of anaphylactic shock and 12 cases of urticaria. In the 2 patients with the most severe anaphylactic shock, evidence of intravascular coagulation was observed, with a diminution of blood clotting factors V, VIII and fibrinogen. In the patient with the mildest anaphylactic shock, as well as the patients with urticaria, no significant changes in the coagulation system were evident, even though the urticaria response indicated allergen sensitivity. The authors concluded that in even severe episodes of anaphylactic shock, in some instances the coagulation system was not activated.

*J. Immunol.*, *123*: 2835—2841 (1979) describes studies conducted involving rabbits, of a basophil-derived platelet activating factor (PAF), which induces the release of a platelet factor 4 (PF4) which is an intrinsic platelet constituent involved in inflammatory reactions. The studies documented that during IgE anaphylaxis the antigen stimulates circulating IgE-sensitized basophils to release PAF, which causes intravascular platelet aggregation.

None of the references described above disclose or suggest a diagnostic test for allergic hypersensitivity involving contacting a patient's blood with an allergen and determining the effect on the time required for hemostasis of the blood. The *J. Immunol.* (115) article describes an increase in the time required for blood coagulation in rabbits following antigen challenge, the *Clin. Res.* article indicates that in known hypersensitive individuals, exposure to the allergen does not correlate with a decrease in blood coagulation factors, even among patients who undergo anaphylactic shock. There is no disclosure of the effect on hemostasis time of the blood.

Summary of the invention

The present invention is directed to a method for determining the allergic sensitivity of a mammal. The method involves the steps of contacting a sample of blood from the mammal with an allergen in the presence of a blood clotting inhibitor and measuring the time required for hemostasis. As a control a sample of blood from the animal is contacted with a blood clotting inhibitor in the absence of the allergen, and the time required for hemostasis is measured. The sensitivity to the allergen is determined by the difference in time required for hemostasis.

Description of the invention

The present invention relates to the determination of allergic sensitivity to specific allergens in allergic individuals. Allergens can be as diverse as pollens, chemicals, insect venom and food, for example, milk, cereal and fish, such as cod, shellfish and haddock. Allergens include antibiotics, penicillin, *Candida albicans*, insulin, ovalbumin, lactalbumin, secale, bermuda grass pollen, timothy grass pollen, orchard grass pollen (cocksfoot grass pollen), combinations of grass pollens, ragweed pollen, ragweed antigen E, birch tree pollen, bee venom, snake venom, horse dander, cat epithelial, dog epithelial, haddock, house dust mite, *Chrysanthemum leucanthemum, Alterraria tenuis*, trypsin, chymotrypsin, dry rot, baker's years, tetanus toxoid, diphtheria toxin, ficin and derivatives thereof. Regardless of the identity of the allergen, it is theorized that the underlying mechanism of allergen response is the same.

When blood vessels in mammals are cut, platelets adhere to exposed collagen and to each other to form aggregates resulting in hemostasis, i.e., the arrest of bleeding. Hemostasis therefore measures the "bleeding time" of a mammal. Hemostasis is a result of a dynamic process involving the interaction of several elements, among them platelets, plasma coagulation factors and the blood vessel walls. Hemostasis can be simulated as described hereinafter.

Formation of a hemostatic aggregate is an early stage of the complex process involved in blood coagulation. Blood coagulation includes the conversion of prothrombin to thrombin and the conversion by thrombin of fibrinogen into fibrin and formation of blood clots. This final formation of a blood clot is referred to as "coagulation". Induction of blood clotting by allergens has been determined to be a relatively inconsistent indicator of allergen sensitivity. Clotting of blood in the present method is in fact undesirable.

The invention involves mixing a blood sample with a clotting inhibitor to prevent blood clotting. The amount of clotting inhibitor required is easily determined by routine testing. It has been determined that 5 International Units of heparin/ml of blood sample is sufficient. The time required for hemostasis is then measured by simulating blood flow, bleeding, and occurrence of hemostasis, in a mammal. In order to determine the effect of the presence of a selected allergen, hemostasis is measured in the presence of the allergen, and in the absence of the allergen, as a control. The allergen can be used as a liquid extract, solid allergenic material, or can be coupled to a carrier which is a water insoluble synthetic matrix, as taught in U.S. Patent No. 3,720,760 described hereinafter. For example, carbohydrate matrices such as agarose and cellulose, paper discs and the walls of polystyrene test tubes can be used as in the inert carrier. A decrease in the time required for hemostasis was found to correlate with allergic sensitivity.

The identity of the clotting inhibitor is not critical. Suitable clotting inhibitors include heparin, calcium citrate, sodium citrate, calcium oxalate, sodium oxalate and ethylenediamine tetraacetic acid.

As described hereinafter, the time required for hemostasis to occur can be determined using apparatus which includes a circuit of plastic tubing having a section of tubing of reduced bore size leading to and from a reservoir. The reduced bore section has a hole of predetermined diameter, e.g., 0.013 inch. A blood sample, mixed with a clotting inhibitor, is placed into the reservoir. When the attached plastic tubing is contacted with peristaltic rollers this circulates the blood in a pulsatile fashion. When the blood reaches the length of smaller bore size, the velocity of the blood increases, forcing blood out of the hole, as well-defined droplets. The time required for hemostasis, due to the formation of a hemostatic aggregate, is determined by measuring the decrease in the rate of droplet formation, or measuring the time required for the hole to become occluded due to the formation of a hemostatic aggregate.

Suitable apparatus for use in the instant claimed method is described in *J. Lab. Clin. Med., 89*(6): 1306—1313 (1977) or is commercially available from Payton Associates, Buffalo, New York 14202. Other methods of measuring hemostatic aggregate formation can be used.

In order to provide an accurate assessment of the correlation between time required for hemostasis and allergenic sensitivity, a series of tests was conducted to compare the method of the present invention with other methods in common use, e.g., the skin prick test, the RAST method and the allergen-induced basophil histamine release.

Comparative methods
1. Skin prick test

Each patient's skin was pricked with a sterile lancet through a drop of glycerinated solution of allergen; an allergic reaction results in symptoms such as reddening of the skin and the formation of a wheal and

# 0 085 362

flare. Skin prick tests were conducted by administering varying amounts of allergen extracts, expressed as protein nitrogen units (pnu) to each patient.

2. RAST test

Blood serum samples from each patient were tested for the presence of antibodies directed against an allergen by the method described in U.S. Patent No. 3,720,760. Briefly, the method involves coupling the allergen, such as a pollen extract, to a water insoluble polymer and contacting the bound allergen with a blood sample. The allergen can be coupled to a water insoluble synthetic matrix by washing the matrix with distilled water and adding cyanogen bromide to the matrix while maintaining the pH at about 11 with NaOH, to "activate" the matrix. The activated matrix is washed with a coupling buffer, e.g., 0.1 M $NaHCO_3$ and 1.0 M NaCl, at a pH of 8.5. The allergen is then added in a suspension of the coupling buffer. If antibodies to the pollen extract are present, the antibodies bind to the allergen. The bound allergen-antibody is then contacted with radiolabelled anti-antibodies to the allergen. If antibodies are present in the blood sample, the labelled anti-antibodies will be bound to the bound allergen-antibody phase. The amount of radiation emitted by the bound allergen-antibody labelled anti-antibody phase will increase with increasing concentration of antibodies in the blood serum sample. There is a correlation between the presence of allergen specific IgE antibodies present and the patient sensitivity to that allergen.

3. Allergen-induced basophil histamine release

The method is similar to that described *J. Lab. Clin. Med., 81*: 291 (1973), and involves obtaining a blood sample from a patient and mixing the blood sample with heparin and a dextran solution, in a round bottom test tube. The erythrocytes are allowed to settle out, leaving a plasma-leukocyte layer. The plasma-leukocyte layer is centrifuged to remove the "buffy" coat, and the supernatant plasma discarded. The cell precipitate present is washed with a buffer solution, the cells challenged with allergen and the histamine release determined. The more sensitive the patient is to the allergen, the greater the amount of histamine released.

Allergy sensitivity diagnosis is considered to be qualitative or at best only semi-quantitative i.e., the tests described above indicate in a relative manner the degree of sensitivity to a suspected allergen.

Example I

Blood samples were obtained from each patient and tested for allergic sensitivity against cocksfoot grass pollen by the method of the present invention, and compared with allergic sensitivity determined by known prior art methods. Cocksfoot grass pollen is available as a lyophilized partially purified extract from Miles Laboratories, Ltd., Buckinghamshire, England, designated Cocksfoot alpha fraction.

Approximately 2 ml of whole blood was mixed with heparin to a final concentration of 5 International Units heparin/ml blood and injected into a circuit of plastic tubing described earlier, and the time required for hemostasis measured as indicated by the formation of a hemostatic aggregate and occlusion of the hole. A second sample of 2 ml of whole blood was mixed with 5 µl of 20 pnu/ml cocksfoot grass allergen and 5 International Units of heparin/ml and injected into the same size circuit of plastic tubing. The time for hemostasis to occur in the presence of the allergen was measured.

Test results obtained are summarized in Table I below.

4

# 0 085 362

TABLE I

Sensitivity to cocksfoot allergen

| Patient | Hemostasis time (min/sec) | | Percent decrease | Skin prick test (PNU/ml) | | | RAST | Basophil sensitivity |
|---|---|---|---|---|---|---|---|---|
| | Control | Cocksfoot allergen | | 5000 | 500 | 50 | | |
| 1 | 11—56 | 7—03 | 40.6 | + | + | + | 3 | +++ |
| 2 | 6—58 | 2—26 | 65.1 | + | + | − | 3 | ++ |
| 3 | 11—29 | 2—07 | 81.5 | + | + | − | 2 | ++ |
| 4 | 8—42 | 7—10 | 17.6 | + | + | − | 2 | NT |
| 5 | 7—03 | 5—06 | 27.6 | + | + | + | 1 | + |
| 6 | 5—17 | 1—41 | 68.1 | + | + | − | 0 | + |
| 7 | 6—53 | 3—59 | 42.1 | − | − | − | 1 | + |
| 8 | 14—01 | 14—33 | Increased | − | − | − | NT | − |

NT—not tested.

For Basophil Sensitivity:— +++=large amount of histamine released at optimum allergen concentration (greater 25 percent of total histamine); ++=medium amount of histamine released at optimum allergen concentration (10—25 percent of total histamine); +=low amount of histamine released at optimum allergen concentration (1—10 percent of total histamine).

| RAST | Class | ng of Specific IgE/ml serum |
|---|---|---|
| undetectable level of allergen specific IgE | 0 | up to 2.4 |
| low level of allergen specific IgE | 1 | up to 4.8 |
| moderate level of allergen specific IgE | 2 | up to 24 |
| high level of allergen specific IgE | 3 | up to 120 |
| very high level of allergen specific IgE | 4 | greater than 120 |

As indicated earlier, allergy diagnosis is considered to be qualitative or semi-quantitative, i.e., various allergy tests can be examined to indicate whether the patient appears to have some sensitivity to a suspected allergen.

Patients 1—7 showed allergic sensitivity to one or more of the skin prick test, RAST or basophil sensitivity test. The above test results also indicate that each of these patients showed a significant decrease in hemostasis time, which correlated with these allergen sensitivity tests. Although patient 7 did not appear to be sensitive to the allergen in the skin prick test, the RAST test and basophil sensitivity indicated some low level of sensitivity. The reduction of hemostasis time also indicated allergen sensitivity.

Based on the research conducted, correlated with the other allergen sensitivity tests described, it was concluded that about a 25 percent reduction of hemostasis time indicated allergen sensitivity.

Patient 8 appeared to be insensitive to cocksfoot pollen, based on the skin prick test and the basophil sensitivity; the hemostasis time showed a slight increase. It was concluded that patient 8 was not sensitive to the allergen.

The above test results indicate satisfactory correlation between cocksfoot allergen sensitivity, indicated by a decrease in time required for hemostasis, and allergen sensitivity measured by one or more tests in clinical use: the skin prick test, RAST and basophil sensitivity.

Example II

A similar procedure to the procedure of Example I was followed except that the allergen used was the allergen involved in sensitivity to house dust mite, which are minute arthropods of the order Acarina. House dust mite allergen used was a lyophilized, partially purified aqueous extract of D. Pteronyssinus, designated Alpha fraction from Miles Laboratories. House dust mite test results obtained are summarized in Table II below.

5

### TABLE II
#### Sensitivity to house dust mite (D. Pteronyssinus) allergen

| Patient | Hemostasis time (min/sec) | | Percent decrease | Skin prick test (PNU/ml) | | RAST | Basophil sensitivity |
|---|---|---|---|---|---|---|---|
| | Control | Mite allergen | | 1500 | 500 | | |
| 9 | 14—01 | 2—32 | 81.9 | + | − | 3 | +++ |
| 10 | 11—29 | 5—03 | 54.1 | + | + | 2 | NT |
| 11 | 2—37 | 1—37 | 38.2 | + | + | 2 | +++ |
| 12 | 8—42 | 2—13 | 74.5 | + | + | 0 | NT |
| 13 | 7—03 | 6—23 | 10.4 | + | − | 0 | NT |
| 14 | 7—34 | 2—25 | 60.6 | + | − | 0 | + |
| 15 | 11—56 | 7—49 | 34.5 | + | − | 0 | NT |
| 16 | 6—59 | 5—09 | 30.6 | − | − | 0 | NT |
| 17 | 2—33 | 2—23 | 6.5 | − | − | NT | NT |
| 18 | 0—36 | 0—37 | Increase | − | − | NT | NT |

NT—not tested. Basophil sensitivity details as in Table I.
RAST details as in Table I.

Patients 9—15 showed allergic sensitivity to one or more of the skin prick test, RAST or basophil sensitivity test. Each of these patients showed a significant decrease in hemostasis time, which correlated with these allergic sensitivity tests. Although patient 16 appeared to be insensitive to house dust mite, based on the skin prick test and the RAST test, hemostasis time decreased sufficiently to indicate possible sensitivity. As indicated earlier, about 25 percent reduction in hemostasis time indicates allergen sensitivity. Patients 17 and 18 were insensitive to the allergen, based on the hemostasis test and the skin prick test.

The above test results indicate satisfactory correlation between the house dust mite sensitivity, indicated by a decrease in time required for hemostasis, and allergen sensitivity measured by one or more tests in clinical use; the skin prick test, RAST and basophil sensitivity.

The above results indicate that the claimed method can be used as a reliable indicator of allergic sensitivity to various allergens.

**Claims**

1. A method for determining the allergic sensitivity of a mammal which comprises the steps of contacting a sample of blood from said mammal with an allergen in the presence of a blood clotting inhibitor, measuring the time required for hemostasis of said sample, contacting a sample of blood from said animal with a clotting inhibitor in the absence of said allergen and measuring the time required for hemostasis in said sample, and determining the difference in time required for hemostasis whereby the sensitivity to said allergen is determined.

2. A method as claimed in claim 1 wherein the allergen is bound to an inert carrier.

3. A method as claimed in claim 1 wherein the allergen is selected from the group consisting of penicillin, *Candida albicans*, insulin, ovalbumin, lactalbumin, secale, bermuda grass pollen, timothy grass pollen, cocksfoot grass pollen, combinations of grass pollens, ragweed pollen, ragweed antigen E, birch tree pollen, bee venom, snake venom, horse dander, cat epithelial, dog epithelial, haddock, cod, house dust mite, *Chrysanthemum leucanthemum, Alternaria tenuis,* trypsin, chymotrypsin, dry rot, baker's yeast, tetanus toxid, diptheria toxin, ficin, and derivatives thereof.

4. A method as claimed in claim 4 wherein the allergen is a grass pollen or house dust mite.

5. A method as claimed in claim 1 wherein the clotting inhibitor is selected from the group consisting of heparin, sodium citrate, calcium citrate, calcium oxalate, sodium oxalate, and ethylenediamine tetraacetate.

6. A method as claimed in claim 6 wherein the clotting inhibitor is heparin.

## 0 085 362

### Patentansprüche

1. Verfahren zur Bestimmung der allergischen Empfindlichkeit eines Säugers, gekennzeichnet durch die folgenden Schritte: Kontaktieren einer Blutprobe von dem genannten Säuger mit einem Allergen in Gegenwart eines Blutgerinnungsinhibitors, Messen der Zeit, die zur Hämostase dieser Probe erforderlich ist, Kontaktieren einer Blutprobe von dem genannten Säuger mit einem Gerinnungsinhibitor in Abwesenheit des genannten Allergens und Messen der Zeit, die zur Hämostase der genannten Probe erforderlich ist, und Ermitteln des Zeitunterschiedes, der zur Hämostase erforderlich ist, wodurch die Empfindlichkeit auf das genannte Allergen bestimmt wird.

2. Verfahren nach Anspruch 1, dadurch gekennzeichnet, dass das Allergen an einen inerten Träger gebunden ist.

3. Verfahren nach Anspruch 1, dadurch gekennzeichnet, dass das Allergen ausgewählt ist aus der Gruppe bestehend aus Penicillin, Candida albicans, Insulin, Ovalbumin, Lactalbumin, Secale, Bermuda-Graspollen, Wiesenliesch-Graspollen, Knäuel-Graspollen, Kombinationen von Graspollen, Kreuzkraut-Pollen, Kreuzkraut-Antigen E, Birkenpollen, Bienengift, Schlangengift, Pferdeschuppen, Katzenepithel, Hunde-Epithel, Schellfisch, Kabeljau, Hausstaubmilbe, Chrysanthemum leucanthemum, Alternaria tenuis, Trypsin, Chymotrypsin, Trockenfäule, Bäckerhefe, Tetranustoxoid, Diphtherietoxin, Ficin, und Derivative derselben.

4. Verfahren nach Anspruch 4, dadurch gekennzeichnet, dass das Allergen Graspollen oder Hausstaubmilbe darstellt.

5. Verfahren nach Anspruch 1, dadurch gekennzeichnet, dass der Gerinnungsinhibitor ausgewählt wird aus der Gruppe bestehend aus Heparin, Natriumcitrat, Calciumcitrat, Calciumoxalat, Natriumoxalat, und Ethylendiamintetraacetat.

6. Verfahren nach Anspruch 5, dadurch gekennzeichnet, dass der Gerinnungsinhibitor Heparin darstellt.

### Revendications

1. Méthode de détection d'une sensibilité allergique chez un mammifère, qui comprend les étapes consistant à mettre un échantillon de sang dudit mammifère en contact avec un allergène en présence d'un inhibiteur de coagulation sanguine, à mesurer le temps nécessaire pour l'hémostase dudit échantillon, à faire entrer un échantillon de sang dudit animal en contact avec un inhibiteur de coagulation en l'absence dudit allergène et à mesurer le temps nécessaire pour l'hémostase dudit échantillon, et à déterminer la différence de temps nécessaire pour l'hémostase, ce qui permet de déterminer la sensibilité audit allergène.

2. Méthode suivant la revendication 1, dans laquelle l'allergène est lié à un support inerte.

3. Méthode suivant la revendication 1, dans laquelle l'allergène est choisi dans le groupe comprenant la pénicilline, Candida albicans, l'insuline, l'ovalbumine, la lactalbumine, le pollen de seigle, le pollen d'herbe des Bermudes, le pollen, de fléole, le pollen de panic pied-de-coq, des mélanges de pollens, de graminées, de pollen d'ambrosie, l'antigène E d'ambrosie, le pollen de bouleau, le venin d'abeille, le venin de serpent, les fragments de poils de cheval, l'épithélium de chat, l'épithélium de chien, le haddock, la morue, l'acarien des poussières de maison, Chrysanthemum leucanthemum, Alternaria tenuis, la trypsine, la chymotrypsine, le carie rouge, la levure de boulangerie, la toxine du tétanos, la toxine de la diphtérie, la ficine et leurs dérivés.

4. Méthode suivant la revendication 3, dans laquelle l'allergène est un pollen de graminées ou l'acarien des poussières de maison.

5. Méthode suivant la revendication 1, dans laquelle l'inhibiteur de coagulation est choisi dans le groupe comprenant l'héparine, le citrate de sodium, le citrate de calcium, l'oxalate de calcium l'oxalate de sodium et un éthylènediamine-tétraacétate.

6. Méthode suivant la revendication 5, dans laquelle l'inhibiteur de coagulation est l'héparine.